# EUROPÄISCHE PATENTSCHRIFT

(11) **EP 3 120 828 B1**
(45) Veröffentlichungstag und Bekanntmachung des Hinweises auf die Patenterteilung: **12.06.2019**
(21) Anmeldenummer: 16001566.5
(22) Anmeldetag: 24.02.2010
(51) Int. Cl.: A61K 8/41, A61Q 15/00, A61K 8/26, A61K 8/28, A61K 8/86, D06F 35/00

(54) **VERWENDUNG VON GELADENEN TENSIDEN ZUR VERMINDERUNG DER TEXTILVERFLECKUNG DURCH ANTITRANSPIRANTIEN**
USE OF CHARGED SURFACTANTS FOR REDUCING TEXTILE STAINING BY ANTIPERSPIRANTS
UTILISATION DE TENSIOACTIFS CHARGES DESTINES A REDUIRE LES TACHES AU MOYEN D'ANTI-TRANSPIRANTS

(30) Priorität: 27.02.2009 DE 102009010665
(43) Veröffentlichungstag der Anmeldung: 25.01.2017
(62) Teilanmeldung aus: 10705820.8
(73) Patentinhaber: Beiersdorf AG, 20253 Hamburg (DE)
(72) Erfinder: Biel, Stefan, D-21077 Hamburg (DE); Miertsch, Heike, D-22459 Hamburg (DE); Urban, Michael, D-22523 Hamburg (DE); Brüning, Stefan, D-40593 Düsseldorf (DE); Kawa, Rolf, D-40789 Monheim (DE); Kühne, Sabine, D-40764 Langenfeld (DE)
(74) Vertreter: Hartmann, Jost

(56) Entgegenhaltungen:
- EP-A1- 0 550 960
- EP-A2- 0 676 193
- EP-A2- 1 027 880
- WO-A1-01/24766
- WO-A1-94/24997
- WO-A1-03/053388
- DE-A1- 2 113 864
- DE-A1- 2 301 829
- DE-A1- 2 410 610
- DE-A1-102006 047 247
- US-A- 5 997 850

## Beschreibung

Die Erfindung umfasst die Verwendung von geladenen Tensiden in antitranspirantwirksamen Zubereitungen zur Verminderung der Fleckenbildung in oder auf der Kleidung und der Verbesserung der Auswaschbarkeit von Flecken aus der Kleidung.

Bei Anwendung von Äntitranspirantien oder Deodorantien reklamieren viele Verbraucher unerwünschte Flecken im Achselbereich in der Kleidung. Es handelt sich dabei häufig um gelbliche Flecken, die auch zu Verkrustungen neigen können. Diese Ablagerungen und Flecken entstehen vor allem durch ein komplexes Zusammenspiel zwischen Produkt, Hautfett, Schweiß und Waschmittel und lassen sich durch herkömmliche Reinigungsverfahren nur schwer entfernen.

Die Flecken können je nach Person unterschiedlich ausgeprägt sein. Eine Ursache sind die in den meisten Deo-AT-Produkten eingesetzten Aluminiumsalze, die als Antitranspirant-wirkstoffe fungieren. Diese hartnäckigen Verfärbungen lassen sich beim Waschen, auch bei Vorbehandlungen mit Fleckenmittel, nicht oder nur schwer vollständig entfernen.

Es gibt zahlreiche Literatur und Patente, die sich mit der Fleckenbildung auf der Haut und der Kleidung und deren Vermeidung bei der Anwendung von Antitranspirantien befassen.

In der EP 1178775 A1 wird die Verwendung von wasserlöslichen Tensiden zur Verbesserung der Abwaschbarkeit der Rückstände von der Haut und Kleidung beschrieben. Es werden Kombinationen von adstringierenden Salzen mit wasserlöslichen, nichtionischen Tensiden beschrieben, die einen raschen Antitranspirantwirkungseintritt und eine hohe Effektivität aufweisen sollen.

Die EP 973492 A1 beschreibt die Verwendung von oberflächenaktiven Substanzen, hauptsächlich nichtionische Emulgatoren, in Antitranspirantien. Die Antitranspirantstiftformulierungen umfassen nichtflüchtige Emollients, ein Träger, z.B. Cyclomethicon, ein Fettalkohol, wie Stearylalkohol, ein Antitransspirantmaterial und ein Tensid. Es wird die Problematik der Bildung von weißen Rückständen auf der Haut und der Kleidung, die sich bei Anwendung von aluminiumhaltigen Antitranspirantien bilden können, beschrieben Hierin wird das Problem des Weißelns der Formulierung durch einen Refraktionsindexabgleich der Bestandteile angegangen.

EP 858317 A1 beschreibt Zubereitungen mit oberflächenaktiven Substanzen mit einem HLB > 10 zur Entfernung der fettigen Rückstände auf der Haut.

EP 696188 A1 beschreibt den Einsatz eines wash-off Agents zur Entfernung der Lipidkomponenten von der Haut, bevorzugt werden dafür Ethoxylate eingesetzt.

Die DE 102008052748, eine frühere Anmeldung der Patentinhaberin, beschreibt, dass in wasserfreien Suspensionen Emulgatoren zur verbesserten Abwaschbarkeit der Formulierung von der Haut eingesetzt werden. Die in der wasserfreien Formel vorteilhaft enthaltenen Strukturgeber können auf der Haut fühlbar wachsige Rückstände hinterlassen. Durch die Anwesenheit von polaren Gruppen an den eingesetzten Emulgatoren wird beim Abwaschen der Formulierung die Affinität zum Wasser erhöht und die Rückstände verschwinden. Dafür eignen sich bevorzugt nichtionische Emulgatoren.

Wünschenswert ist es kosmetische Antitranspirantien zur Verfügung zu stellen, die eine verringerte Fleckenbildung in der Kleidung aufweisen und vor allem die nachträgliche Auswaschbarkeit verbessern helfen.

Zusätzlich dürfen diese kosmetischen Zubereitungen keinerlei Instabilitäten aufweisen und sollten einfach zu formulieren sein.

Wesentlicher Aspekt bei der Formulierung kosmetischer Antitranspirantien ist deren Hautverträglichkeit, so dass neben der Aufgabe der Fleckvermeidung oder verbesserten Auswaschbarkeit auch die Hautverträglichkeit zu berücksichtigen ist.

Bekannt sind des Weiteren waschaktive Substanzen, wie beispielsweise kationische Tenside insbesondere quartäre Ammoniumverbindungen. Eine waschaktive Substanz findet in Waschmitteln, Spülmitteln, Shampoos, Duschgels Verwendung und bezeichnet den Anteil der Formulierung, der die Wasch- oder Reinigungsleistung beeinflusst. Waschaktive Substanzen erhöhen die "Löslichkeit" von Fett- und Schmutzpartikeln in Wasser, die in der Wäsche oder am Körper haften. Sie können natürlichen oder synthetischen Ursprungs sein. Sie werden nach der Art ihrer Ladung in anionisch, kationisch, ampholytisch oder nichtionisch unterschieden.

In der DE 10322059 A1 werden Haarbehandlungsmittel mit Styling-Eigenschaften beschrieben, die quartäre Ammoniumverbindungen als kationisches Tensid umfassen.

In der DE 102007028508 werden waschaktive Substanzen zur besseren Abwaschbarkeit der kosmetischen Mittel von der Haut beschrieben. Quartäre Ammoniumverbindungen gelten dabei auch als antimikrobieller Wirkstoff und Konditionierungsmittel bzw. Weichmacher.

DE2113864 offenbart Antischweissmittelaerosole, in denen durch Zusatz von nichtionischen Tensiden (Pluronics) die Fleckenbildung auf Textilien herabgesetzt wird.

In der DE 102005029386 werden Wirkstoffkombinationen aus Glycopyrroniumbromid und einem oder mehreren Hydrokolloiden beschrieben, die darüber hinaus kationische Tenside enthalten können.

DE 10321138 offenbart zweiphasige Antitranspirantprodukte, wobei die Ölphase u. a. einen nichtionischen, kationischen, zwitterionischen oder amphoteren Emulgator enthält.

In der DE 10309180 werden quartäre Ammoniumverbindungen in Haut- und Haarpflegemitteln zur Körperpflege als kationisches Tensid eingesetzt.

DE 102006037113 offenbart kosmetisches Haarbehandlungsmittel mit Antischuppenwirkstoffen, wobei u.a. Laureth-7 Citrate sowie weitere kationische Tenside enthalten sein können. Antitranspirantmittel werden nicht offenbart.

US 4477431 offenbart fließfähige, wasserfreie Zubereitung mit 35-70 Gew.% Pulver, 1-20 Gew.% anorganischem Verdicker, 19-45 Gew.% Öl und 1-10 Gew.% Surfactants, die nichtionisch und/oder kationisch sein können.

EP 2082724 offenbart ein antimikrobielles System mit einem kationischen Tensid, welches ein Ethylester vom Lauramid von Argininhydrochlorid (LAE) darstellt.

EP 1183003 offenbart Antitranspirantzubereitung mit feinteiligen Aluminium-, Zirkonium- oder Zinksalzen, wobei die Teilchen mit einem wasserlöslichen nichtionischen, zwitterionischen oder kationischen Tensid imprägniert oder umhüllt sind.

EP 676193 offenbart Zubereitung mit einem Wirkstoff, einem "borate crosslinker", einem Tensid und Wasser. Der Wirkstoff kann u.a. Aluminiumsalz sein und das Tensid ist vorzugsweise nichtionisch. Die Tenside verhindern, dass das Produkt hart und spröde wird.

EP 10163247 offenbart die Schaumapplikation eines Deo- oder AT-Wirkstoffs aus einem Schaumdispenser (Pumpe oder Treibgas) aus einer flüssigen, schäumenden Tensidlösung, die aus Wasser, mindestens ein schäumendes Tensid, Öl, Parfüm, mindestens ein nichtionischer Lösungsvermittler für das Parfüm und einem Deo- oder AT-Wirker besteht.

Das unter dem Handelsnamen VARISOFT® PATC der Firma Degussa (Evonik) vertriebene Haarkonditionierungsmittel führt als eine der herausragenden Eigenschaften die Reduktion des Auswaschens chemischer Haarfarben beim Einsatz von Haarshampoos auf.

D.h. bei Einsatz von VARISOFT® PATC wird ein verlängertes Verbleiben der Farbe auf dem Haar erwartet.

Der dargestellte vorliegende Stand der Technik konnte den Weg zur vorliegenden Erfindung nicht vorzeigen.

Die Erfindung umfasst antitranspirantwirksame Zubereitungen umfassend ein oder mehrere geladene Tenside entsprechend Anspruch 1.

Als Zubereitungen sind sowohl kosmetische als auch dermatologische Formulierungen geeignet.

Die Zubereitungen umfassend ein oder mehrere geladene Tenside vermindern oder vermeiden die Fleckenbildung in oder auf der Kleidung und verbessern die Auswaschbarkeit von Flecken, die durch die Zubereitung selber mit verursacht wurden, aus der Kleidung.

Bevorzugt wird das Gewichtsverhältnis Antitranspirantwirkstoff zu Tensid im Bereich 1 : 1 bis 30 : 1, vorzugsweise 2 : 1 bis 20 : 1, ganz besonders bevorzugt 3 : 1 bis 8 : 1 gewählt..

Wenn als geladene Tenside sowohl mindestens ein kationisches als auch mindestens ein anionische Tensid erfindungsgemäß enthalten sind, beträgt das Gewichtsverhältnis zwischen kationischen und anionischen Tensiden vorteilhaft 10 : 1 bis 1 : 3, vorzugsweise 6 : 1 bis 1 : 2.

Erfindungsgemäß ist somit insbesondere die Verwendung eines oder mehrerer geladener Tenside in kosmetischen oder dermatologischen Zubereitungen umfassend ein oder mehrere antitanspirantwirksame Stoffe zur Verminderung oder Vermeidung der durch die Zubereitung bedingten Verfleckung auf der Kleidung und zur verbesserten Auswaschbarkeit dieser Flecken. Vornehmlich handelt es sich um Zubereitungen umfassend ein oder mehrere Antitranspirantien, insbesondere solche auf Aluminiumbasis.

Unter Verminderung bzw. Vermeidung der Fleckenbildung auf Textilien vor und insbesondere nach der Wäsche wird erfindungsgemäß der verringerte b-Wert verstanden,
der photometrisch mittels der Farbmaßzahlen im CIE- L*a*b-Farbraum ermittelt wird, und der gegenüber den b-Werten der Textilie, befleckt mit einer Zubereitung mit Antitranspirantmittel aber ohne erfindungsgemäße Tenside, gemessen wird.

Durch ein Paneltest ist gezeigt worden, dass die Fleckenbildung der erfindungsgemäßen Zubereitung in oder auf der Kleidung grundsätzlich vermindert ist bzw. die Kleidung weniger gelblich verfleckt ist. Dies wird nachfolgend erläutert.

Um den Schweißgeruch über einen längeren Zeitraum zu unterdrücken, ist der Einsatz kosmetischer Zubereitungen unerlässlich. Den üblichen kosmetischen Deodorantien liegen unterschiedliche Wirkprinzipien zugrunde, die auch kombiniert werden können: Zum einen werden Deowirkstoffe eingesetzt, die das Wachstum der den Schweißgeruch verursachenden Bakterien unterdrücken. Zu diesen keimhemmenden (bakteriostatischen) Mitteln zählen beispielsweise Triclosan, Chlorhexidin oder die natürlich vorkommenden Verbindungen wie Farnesol und Phenoxyethanol.

Zum anderen werden Antitranspirantien eingesetzt, welche die Schweißabsonderung durch Blockierung der Schweißdrüsenausgänge behindern. In den weitaus meisten Antitranspirantien kann durch Adstringentien - vorwiegend Aluminiumsalze wie Aluminiumhydroxychlorid (Aluminiumchlorohydrat) oder Aluminium/Zirkoniumsalze - die Bildung des Schweißes reduziert werden.

Auch die Kombination von Adstringentien mit antimikrobiell wirksamen Stoffen in ein- und derselben Zusammensetzung ist gebräuchlich. Ferner werden Parfümstoffe zur Überdeckung des Schweißgeruches eingesetzt.

Bekannt sind weitere antitranspirantwirksame Verbindungen, wie beispielsweise 4-[(2-cyclopentyl-2-hydroxyphenylacetyl)oxy]-1,1-dimethyl-piperidiniumbromid.

Bekannt und gebräuchlich sind neben den flüssigen Desodorantien wie Zerstäuber und Roll-on auch feste Zubereitungen, beispielsweise Deo-Stifte ("Sticks"), Puder, Pudersprays, Intimreinigungsmittel usw.

Als Antitranspirantwirkstoff lassen sich vorteilhaft aktivierte saure Aluminium- und/oder Aluminium/Zirkoniumsalze in wässriger Lösung einarbeiten. Hierbei beziehen sich die beschriebenen Konzentrationsbereiche auf die so genannten Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe als Puffer wird hier üblicherweise Glycin verwendet).

Die nachfolgende Auflistung vorteilhaft einzusetzender Antitranspirant-Wirker soll in keiner Weise einschränkend sein:
Aluminium-Salze (der empirischen Summenformel [Al₂(OH)ₘClₙ], wobei m+n=6):
   - Aluminiumchlorhydrat [Al₂(OH)₅Cl] x H₂O
      Standard Al-Komplexe: Locron L, Locron LIC, Locron LIF (Clariant), Chlorhydrol (Reheis), ACH-303 (Summit), Aloxicoll L (Giulini).
      Aktivierte Al-Komplexe: Reach 501 (Reheis), Aloxicoll 51L
   - Aluminiumsesquichlorhydrat [Al₂(OH)_{4,5}Cl_{1,5}] x H₂O
      Standard Al-Komplexe: Aloxicoll 31L (Giulini), Westchlor 186 (Westwood Chemicals) Aktivierte Al-Komplexe: Reach 301 (Reheis)
   - Aluminiumdichlorhydrat [Al₂(OH)₄Cl₂] x H₂O
Aluminium-Zirkonium-Salze:
   - Aluminium/Zirkonium Trichlorhydrex Glycin [Al₄Zr(OH)₁₃Cl₃] x H₂O x Gly Standard Al/Zr-Komplexe: Rezal 33GC (Reheis), AZG-7164 (Summit)
   - Aluminium/Zirkonium Tetrachlorhydrex Glycin (GLY) [Al₄Zr(OH)₁₂Cl₄] x H₂O x Gly Standard Al/Zr-Komplexe: Rezal 36, Rezal 36G, Rezal 36 GC (Reheis), AZG-368 (Summit), Zirkonal L435G (Giulini), Westchlor ZR 35 BX5, Westchlor ZR 41 (Westwood Chemicals)
   - Aluminium/Zirkonium Pentachlorhydrex Glycin [Al₈Zr(OH)₂₃Cl₅] x H₂O x Gly
      Standard Al/Zr-Komplexe: Rezal 67 (Reheis), Zirkonal L540, Zirkonal L530 PG (Giulini), Westchlor ZR 80B (Westwood Chemicals)
   - Aluminium/Zirkonium Octachlorhydrex Glycin [Al₈Zr(OH)₂₀Cl₈] x H₂O x Gly: Westchlor ZR 82B
   - Reach AZP - 908 SUF activated Aluminum Zirkonium Tetrachlorohydrex Gl
   - Reach AZZ - 902 SUF activated Aluminum Zirkonium Trichlorohydrex Glyc

Ebenso vorteilhaft können aber auch puffer-freie Aluminium/Zirkonium-Salze eingesetzt werden.

Die Antitranspirant-Wirkstoffe aus den zuvor geschilderten Gruppe der adstringierende AT-Mittel, den klassischen AT-Mittel werden in den erfindungsgemäßen Formulierungen in einer Menge von 0,05 bis 40 Gew.%, vorzugsweise von 0,1 bis 20 Gew. %, bezogen auf die Gesamtmasse der Zubereitung, d.h. inklusive ggf. vorhandener Treibgase, eingesetzt.

Nachteilig bei der Verwendung von Antitranspirantien, insbesondere Aluminiumsalzen, ist, wie zuvor ausgeführt, die Bildung von Rückständen in oder auf der Kleidung, die die Kleidung in unschöner Weise verfärben können.

Unter Textilverfleckung werden Verfleckungen, insbesondere im Achselbereich, verstanden. Es handelt sich um Flecken, die nach dem Tragen und/oder Waschen in der Kleidung verbleiben und mit zunehmendem Alter des Kleidungsstückes intensiver werden können. Diese Flecken sind nicht die als "Weißeln" bezeichneten Rückstände auf der Haut oder Kleidung zu verstehen.

Es handelt sich erfindungsgemäß vielmehr um meist gelbliche Verfleckungen, die entstehen, wenn das kosmetische Produkt oder Bestandteile davon beim Schwitzen zusammen mit den Körpersekreten der Achsel auf die Textilie gelangt. Durch das Waschen der Textilie wird ein Teil dieser Ablagerungen ausgewaschen, ein anderer Teil verbleibt als Rückstand auf dem Textil.

Erfindungsgemäß vermag die Zubereitung nicht die Bildung jeglicher Flecken, die sich auf einem Kleidungsstück durch vielfältige Ursachen bilden können, vermindern oder verhindern. Jedoch kann die erfindungsgemäße Zubereitung die Bildung derjenigen Flecken, die durch die Zubereitung selber mit verursacht werden, insbesondere diejenigen durch Antitranspirantstoffe, vermindern oder verhindern sowie deren Auswaschbarkeit verbessern.

Als Maß der Verbesserung bzw. Verminderung wird dabei der Unterschied zur Fleckenbildung bzw. dessen Auswaschung bei der Anwendung der erfindungsgemäßen Zubereitung und der Zubereitung ohne geladene Tenside definiert.

Aber auch grundsätzlich beurteilen unabhängige Beobachter (Panel), dass die Flecken der erfindungsgemäßen Zubereitungen auf der Kleidung gar nicht oder zumindest weniger gelb ausfallen. Eine grundsätzliche Fleckenverminderung ist damit gegeben.

Erfindungsgemäß nicht gemeint sind Verfleckungen, die als weiße Flecken im Falle des direkten Kontaktes eines Deodorants oder Antitranspirants mit dem Stoff entstehen. Dies sind eher die weißliche Ablagerungen der Rezepturbestandteile, z.B. Aluminiumsalze. Diese Flecken sind leicht vermeidbar, wenn dem Produkt vor Anlegen der Kleidung die Möglichkeit zum Trocknen gegeben wird. Diese weißen Rückstände sind in der Regel mechanisch (Bürsten) oder durch Waschen zu entfernen. Diese Problematik des "Weißelns" ist im Stand der Technik ausführlich beschrieben und es werden darin Lösungsansätze geboten. Erfindungsgemäß geht es insbesondere um die bekannte gelbliche Verfleckung von Antitranspirantien in oder auf der Kleidung, nachdem das Kleidungsstück gewaschen wurde. Erfindungsgemäß setzt hier auch der Lösungsgedanke der verbesserten Auswaschbarkeit an. Der gelb-Wert des Fleckes wird daher insbesondere über den b-Wert definiert, der photometrisch mittels der Farbmaßzahlen im CIE- L*a*b-Farbraum ermittelt werden kann. Bevorzugt bezieht sich die erfindungsgemäße Verwendung der Fleckenvermeidung bzw. verbesserten Auswaschbarkeit auf Textilien, die Baumwolle enthalten oder aus Baumwolle bestehen.

Erfindungsgemäß werden den kosmetischen Zubereitungen dazu ein oder mehrere geladene Tenside zugesetzt.

Tenside sind Substanzen, die die Oberflächenspannung einer Flüssigkeit oder die Grenzflächenspannung zwischen zwei Phasen herabsetzen und die Bildung von Dispersionen ermöglichen oder unterstützen. Tenside bewirken, dass zwei eigentlich nicht miteinander mischbare Flüssigkeiten, wie zum Beispiel Öl und Wasser, dispergiert werden können.

Des Weiteren werden Tenside als amphiphile Stoffe beschrieben, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO-, -OSO₃²⁻, -SO₃⁻, während die hydrophoben Teile in der Regel unpolare Kohlenwasserstoffreste darstellen. Tenside werden im Allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quatären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung.

Typisch für nichtionische Tenside sind Polyether-Ketten. Unter nichtionischen Emulgatoren werden grenzflächenaktive Substanzen verstanden, die in wässriger Lösung keine Ionen bilden. Die Hydrophilie solcher nichtionischer Emulgatoren wird durch den Anteil der polaren Gruppen im Molekül erreicht. Zu den nichtionischen Emulgatoren gehören Fettalkohole (z. B. Cetyl- oder Stearylalkohol), partielle Fettsäureester mehrwertiger Alkohole mit gesättigten Fettsäuren (z. B. Glycerolmonostearat), partielle Fettsäureester mehrwertiger Alkohole mit ungesättigten Fettsäuren (z. B. Glycerolmonooleat, Pentaerythritolmonooleat), ferner Polyoxyethylenester von Fettsäuren (z. B. Polyoxyethylenstearat), Polymerisationsprodukte aus Ethylenoxid und Propylenoxid an Fettalkoholen (Fettalkoholpolyglycolether) oder Fettsäuren (Fettsäureethoxylate).

Die nichtionischen Tenside oder Emulgatoren zählen erfindungsgemäß nicht zu den geladenen Tensiden.

Als geladene Tenside sind erfindungsgemäß auch keine Tenside zu verstehen, die als Emulgator in der Rezeptur fungieren.

Tenside als Emulgatoren bewirken, dass zwei nicht miteinander mischbare Flüssigkeiten (zum Beispiel Öl in Wasser) sich zu einer Emulsion vermengen können. Aufgrund des amphiphilen Charakters dringen sie mit ihrem fettlöslichen Teil in das Öl ein. Durch den hydrophilen Teil kann das nun durch Rühren entstandene Öltröpfchen in der wässrigen Umgebung dispergiert" werden. Emulgatoren haben primär keinen waschaktiven, tensidischen Charakter.

Erfindungsgemäßen sind also die nichtionischen Tenside oder Emulgatoren nicht unter dem Begriff der geladenen Tenside zu verstehen.

Es wurde nun überraschend gefunden, dass geladene Tenside, d. h. anionische, kationische oder amphotere Tenside, vorzugsweise kationische und/oder anionische Tenside, erfindungsgemäss enthaltend bestimmte quartäre Ammoniumverbindungen der Formel (I) gemäss Anspruch 1, sogenannte Quats, in kosmetischen Zubereitungen zu einer signifikanten Fleckreduzierung führen, die ansonsten üblicherweise von dem kosmetischen Produkt in der Kleidung hervorgerufen worden wären.

Als bevorzugte quartäre Ammoniumverbindung wird Palmitamidpropyltrimoniumchloride, Handelsname Varisofl® PATC, eingesetzt.

Varisoft® PATC besteht aus 60% Palmitamidopropyltrimoniumchloride in 40% Propylenglykol als Lösemittel. Alternativ kann auch eine Mischung von Palmitamidpropyltrimoniumchlorid und einem Fetalkohol (Ceterayl Alcohol) eingesetzt werden, Handelsname Tego Care CE 40. Neben quartären Ammoniumverbindungen, den Quats, werden weiter bevorzugt auch anionische Tenside, vorzugsweise auf Basis von Carbonsäuren und deren Derivate wie Carbonsäure-Ester als erfindungsgemäßes geladenes Tensid verwendet, insbesondere ethoxylierte Carbonsäure-Ester, hier ganz besonders das Laureth-7 Citrat.

Quartäre Ammoniumverbindung ist die Bezeichnung für organische Ammonium-Verbindungen mit quartären Stickstoff-Atomen. Sie werden durch Umsetzung tertiärer Amine mit Alkylierungsmitteln, wie z. B. Methylchlorid, Benzylchlorid, Dimethylsulfat, Dodecylbromid, aber auch Ethylenoxid hergestellt. In Abhängigkeit von dem eingesetzten tertiären Amin unterscheidet man drei Gruppen: 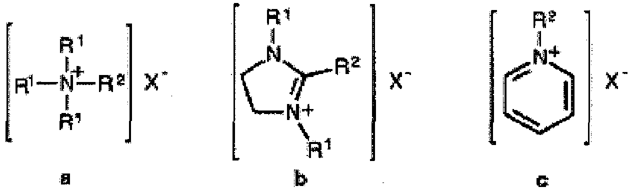 Darin bedeutet: R¹ = CH₃, R² = C₈₋₁₈, X = Halogen
a) Lineare Alkylammonium-Verbindungen, b) Imidazolinium-Verbindungen, c) Pyridinium-Verbindungen

Erfindungsgemäße Formulierungen enthalten mindestens eine Verbindung der Formel (I) 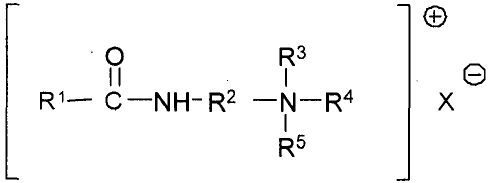
R1 steht für eine gesättigte oder ungesättigte, lineare oder verzweigte Alkyl- oder Arylgruppe mit 8 bis 24 C-Atomen,
R2 steht für eine gesättigte oder ungesättigte, lineare, verzweigte oder cyclische, substituierte oder nicht substituierte Alkylengruppe mit 1 bis 10 C-Atomen und 0 bis 5 Hydroxygruppen,
R3, R4, R5 stehen unabhängig voneinander für Wasserstoff, eine C1-C4-Alkylgruppe, eine C5- oder C6-Cycloalkylgruppe, eine Arylgruppe oder eine C2-C4-Hydroxyalkylgruppe, und X- steht für ein monovalentes Anion
Erfindungsgemäß bevorzugt sind solche Verbindungen nach Formel (I), in denen der Rest R1 für eine gesättigte, lineare C10-C18-Alkylgruppe steht.

Erfindungsgemäß bevorzugt sind auch solche Verbindungen nach Formel (I), in denen der Rest R2 für die Gruppe -CH2CH2CH2- steht.

Weiterhin sind solche Verbindungen nach Formel (I) erfindungsgemäß bevorzugt, in denen die Reste R3, R4 und R5 jeweils für Alkylgruppen, beispielsweise für Methyl-, Ethyl-, Propyl-, Isopropyl- und Butylgruppen stehen. Insbesondere bevorzugt sind Methylgruppen.

Bevorzugt sind solche Verbindungen nach Formel (I), bei denen das monovalente Anion X-für Halogenid, beispielswiese Chlorid oder Bromid oder für eine der beiden Gruppen CH3-O-SO3 (Methosulfat) oder CH3CH2-O-SO3 (Ethosulfat) steht.

Erfindungsgemäß bevorzugt werden lineare Alkylammoniumverbindungen eingesetzt. Bevorzugte quartäre Ammoniumverbindungen sind Ammoniumhalogenide, insbesondere Chloride und Bromide.

Zu den erfindungsgemäßen quartäre Ammoniumverbindungen zählen keine polymeren quartären Ammonium-Verbindungen, da diese zu einer Verstärkung der Verfleckungen führen können.

Zudem sind die Polymere nicht als geladene Tenside zu bezeichnen.

Insbesondere bevorzugte Verbindungen nach Formel (I) sind im Sinne der Erfindung Palmitamidopropyltrimonium Chloride, wie es beispielsweise unter dem Handelsnamen Varisoft® PATC von der Firma Evonik im Handel erhältlich ist, Behenamidopropyl Ethyldimonium Ethosulfate, wie es beispielsweise unter dem Handelsnamen Mackernium BAPDES von der Firma Mclntyre im Handel erhältlich ist, Stearamidopropyl Trimonium Methosulfate, wie es beispielsweise unter dem Handelsnamen Catigene SA-70 von der Firma Stepan Company erhältlich ist und/oder Undecylamidopropyltrimonium Methosulfate, wie es beispielsweise unter dem Handelsnamen Rewocid UTM 185 von der Firma Evonik im Handel erhältlich ist.

Bevorzugtes anionisches Tensid ist Laureth-7 Citrat. Laureth-7 Citrat ist als PLANTAPON LC 7 (Cognis) erhältlich. Es ist ein anionisches, Tensid, ein Ester aus Laureth-7 und Zitronensäure.

Die geladenen Tenside, insbesondere quartäre Ammoniumverbindungen werden vorteilhaft zu einem Anteil von 0,1 bis 5 Gew.%, vorzugsweise 0,5 bis 3 Gew.% Aktivgehalt, d.h. ohne Lösemittel wie Propylene Glycol, bezogen auf die Gesamtmasse der Zubereitung, eingesetzt.

Es hat sich überraschenderweise herausgestellt, dass insbesondere ein bestimmtes Gewichtsverhältnis von Antitranspirantwirkstoff, insbesondere Aluminiumsalzen, zu geladenem Tensid zu einer signifikanten Fleckenverminderung bzw. Auswaschbarkeit beiträgt.

Das Verhältnis Antitranspirantwirkstoff zu Tensid beträgt vorteilhaft 1 : 1 bis 30 : 1, vorzugsweise 2 : 1 bis 20 : 1, ganz besonders bevorzugt 3 : 1 bis 8 : 1.

Bei der Produktform eines Aerosolsprays beziehen sich die Verhältnisse auf die Wirkstofflösung (ohne Treibgas).

Wenn als geladene Tenside sowohl kationische als auch anionische Tenside erfindungsgemäß enthalten sind, beträgt das Gewichtsverhältnis zwischen kationischen und anionischen Tensiden vorteilhaft 10 : 1 bis 1 : 3, vorzugsweise 6 : 1 bis 1 : 2.

Vorzugsweise sind die Gewichtsanteile an Antitranspirantwirkstoffen, insbesondere Aluminiumchlorohydrate, im Bereich von 1 bis 25 Gew.%, insbesondere 5 und 20 Gew.% zu wählen.

Vorteilhaft werden kationische Tenside und anionische Tenside dann insbesondere im Verhältnis 1:1 gewählt.

Interessanterweise wird durch den Zusatz an geladenen Tensiden, insbesondere an quartären Ammoniumverbindungen, insbesondere an Palmitamidopropyltrimoniumchlorid, zu Zubereitungen, umfassend Antitranspirantien, insbesondere Aluminumverbindungen, die Textilverfleckung signifikant verringert.

Insbesondere Quats mit mindestens einer langen Alkylgruppe haben eine Funktion als waschaktive Substanz und werden z. B. als kationische Tenside in Produkten wie Weichspülern eingesetzt. Sie können auch eine desinfizierende Wirkung haben. Weiterhin werden Quats in Haarpflegemitteln eingesetzt. Sie verbessern die physikalischen Eigenschaften wie Kämmbarkeit, Glanz und antistatisches Verhalten des Haares.

Wie im Stand der Technik zu Varisoft® PATC ausgeführt, verlängern diese Substanzen vermeintlich das Verbleiben von Stoffen, wie Farbe auf den Haaren.

Erfindungsgemäß überraschend hat sich nun herausgestellt, dass die geladenen Tenside, wie quartären Ammoniumverbindung, vornehmlich Palmitamidopropyltrimoniumchloride, die Auswaschbarkeit von Flecken verbessern, was der eigentlichen Eigenschaft der Quats gemäß Produktinformation wiederspricht.

Die Verwendung der geladenen Tenside, insbesondere quartäre Ammoniumverbindungen, in kosmetischen Zubereitungen zur Vermeidung oder Verminderung der Fleckenbildung verursacht durch die kosmetische Zubereitung in oder auf Textilien sowie der verbesserten Auswaschbarkeit dieser Flecken, insbesondere aus Baumwolltextilien, ist damit predestiniert.

Erfindungsgemäß überraschend ist auch, dass der Einsatz von quartären Ammoniumverbindungen trotz ihrer Elektrolyteigenschaften keine Stabilitätsprobleme bereiten und somit für die kosmetischen Zubereitungen und Antitranspirantien gängige Formelsystem verwendet werden können.

Bei Einarbeitung der geladenen Tenside ist deren Löslichkeit in den vorhandenen Formelbestandteile zu beachten.

Vorteilhaft sollte daher ein kosmetisch akzeptables Lösemittel vorliegen, z. B. Wasser, Öle und Alkohole. Besonders bevorzugt sind Guerbet-Alkohole, insbesondere Octyldodecanol.

Somit ist eine Einarbeitung von beispielsweise Varisoft® PATC in W/O Emulsionen und wasserfreien Aerosolen einfach möglich, wenn man es vorher in Octyldodecanol löst.

Weiterer wesentlicher Aspekt der erfindungsgemäßen Zubereitungen ist deren weiterhin gesicherte Hautverträglichkeit, die durch den Zusatz an geladenen Tensiden, insbesondere den quartären Ammoniumverbindungen, keinerlei Einbußen befürchten lassen.

Neben der Vermeidung bzw. Verminderung der Fleckenbildung in oder auf der Kleidung ist es überraschenderweise auch gelungen, dass sich die Flecken, die sich nach dem Waschen in der Kleidung gebildet haben, aufgrund des Zusatzes an geladenen Tensiden in der Zubereitung deutlich schwächer erscheinen als ohne den Zusatz an geladenen Tensiden. Zum Nachweis der verbesserten Auswaschbarkeit und verringerter Fleckenbildung wurden folgende Vergleichsversuche in Form von Probandentests (Panel) durchgeführt.

### Ablauf der Untersuchungen:

- Jeder Proband bekommt zwei gleiche Produkte, die sich lediglich im Zusatz des geladenen Tensids (hier: Palmitamidopropyltrimoniumchlorid) unterscheiden. Ein Produkt wird unter der linken, das andere unter der rechten Achsel angewendet.
- Die Produkte werden während des gesamten Testzeitraums angewendet
- Jeder Proband bekommt ein weißes T-Shirt aus Baumwolle.
- Das T-Shirt wird einen Tag getragen und anschließend gewaschen und getrocknet: Waschbedingungen: 60°C Haushaltswaschmaschine, Pulverwaschmittel
- Die gewaschenen T-Shirts werden photometrisch auf eine Fleckbildung im Achselbereich untersucht (Ermittlung der Farbmaßzahlen im CIE-L*a*b-Farbraum)
- Die Farbwerte ergeben sich aus der Differenz der Werte im Achselbereich und der Werte eines Referenzareals (unverfleckter Bereich z. B. Schulter oder Rücken)
- Insgesamt wird mindestens 1 Trage- und Waschzyklus durchgeführt, bevorzugt ist die Durchführung von mindestens 3 - 6 Trage- und Waschzyklen

### Auswertung der Farbwerte

- dL-Wert: weiß bis grau; ein negativer Wert bedeutet eine Vergrauung des T-Shirts
- da-Wert: rot bis grün; ein negativer Wert bedeutet eine Verstärkung im grünen Bereich
- db-Wert: gelb bis blau; ein positiver Wert bedeutet eine Verstärkung im gelben Bereich

**Zusammensetzung der Testprodukte**

| | Produkt ohne | Produkt mit Palmitamidopropyltrimoniumchlorid |
|---|---|---|
| Aluminiumchlorhydrat | 10,0 | 10,0 |
| Isoceteth-20 | 4,8 | 4,8 |
| Dicaprylylether | 3,0 | 3,0 |
| Glycerylisostearat | 2,4 | 2,4 |
| PEG-150 Distearat | 0,7 | 0,7 |
| Butylenglykol | 3,0 | 3,0 |
| Varisoft ® PATC | | 3,0 |
| Parfüm | 1,0 | 1,0 |
| Wasser | 75,1 | 72,1 |
| Farbwerte nach 6 Zyklen | | |
| dL | -2,4 | -1,3 |
| da | -0,6 | -0,2 |
| db | 3,6 | 2,1 |

Die Vergleichszubereitung ohne geladenes Tensid führte zu einer geringeren Auswaschbarkeit, d.h. die Flecken waren im Vergleich zum erfindungsgemäßen Produkt grauer (Differenz L-Wert -1,1), grüner (Differenz a-Wert -0,4) und vor allem gelber (Differenz b-Wert 1,5).

Die Farbwerte wurden aus allen Tests mit allen (7) Probanden zusammengeführt und gemittelt.

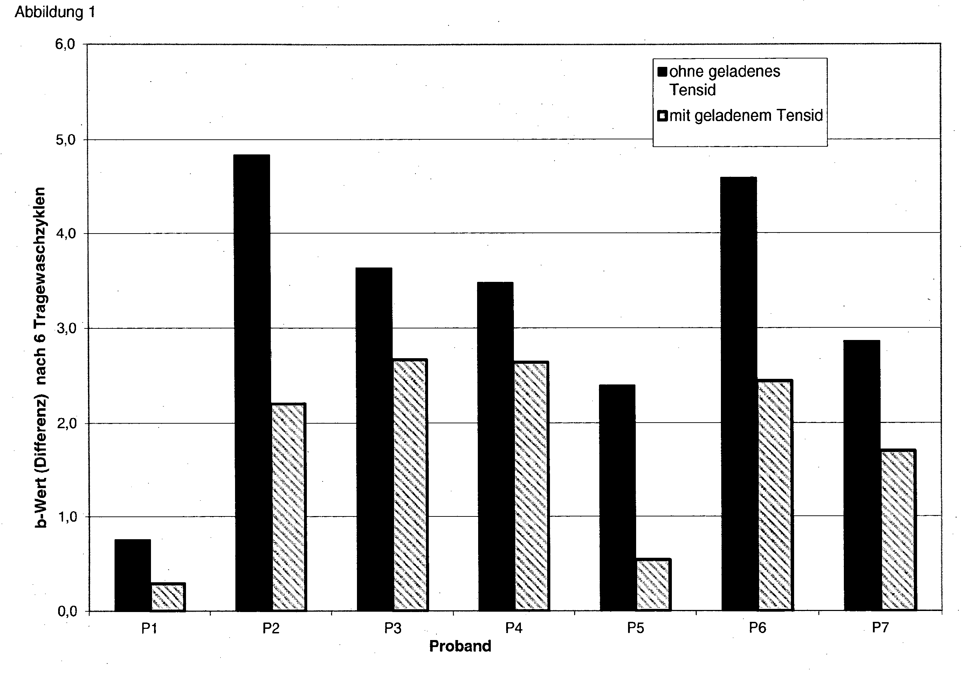

Des Weiteren wurden die Flecken visuell beurteilt. Auf einer Skala von 0 bis 5 haben die Probanden die Fleckbildung beurteilt, wie stark sich ein Fleck im Achselbereich ausgebildet hat. Ein Wert von 0 bedeutet keinen Fleck, ein Wert von 5 bedeutet ein starker Fleck. Im Mittelwert wurde die Fleckintensität für ein Produkt ohne geladenes Tensid mit 3,7 und die mit geladenem Tensid mit 2,7 bewertet
In der Abbildung 1 ist am Beispiel des b-Wertes (gelb) der Unterschied für die Formulierungen mit und ohne geladenes Tensid für 7 einzelne Probanden nach 6 Trage- und Waschzyklen dargestellt. Bei allen Probanden führt das erfindungsgemäße Produkt zu einer geringeren Gelbfärbung.

Als Beleg der verbesserten Auswaschbarkeit und verminderten Fleckenbildung der erfindungsgemäßen Zubereitungen wurden weitere in-vitro und in-vivo Untersuchungen durchgeführt.

### In vitro Methode zur Bestimmung der Fleckintensität

### Materialien:

Textil: weiß, 100% Baumwolle (Jersey-Qualität)
Testsubstanzen/Medien
a.) Sebum: Sebum nach BEY
b.) Schweiß: Humanschweiß (künstl. Achselschweiß)
c.) Vergleichsprodukte
Das Auftragen des Schweißes ist optional.
Wasserqualität: Leitungswasser (vorzugsweise mittelhart bis hart)
Waschmittel: handelsübliche Waschmittel, z. B. Spee Megaperls
Waschmaschine: Linitest+ von ATLAS
Das Sebum bewirkt, dass der Fleck einen vergleichbaren Gelbanteil bekommt. Ohne Sebum werden die Flecken eher grau. Eine derartige Methode ist in der Literatur bereits beschrieben: The Trouble with Stains. SPC July page 25-28; Materialien wie Sebum nach BEY sind beispielsweise als Standard-Testschmutz bei der WfK-Testgewebe GmbH erhältlich.

### Auftragsmengen:

a.) Zubereitung: 1 g auf einem Areal von 15 cm x 2,5 cm der Textilie, entspricht 27 mg/cm²
b.) Humanschweiß: 0,5 g auf einem Areal von 15 cm x 2,5 cm, entspricht 13 mg/cm²
c.) Sebum: 0,25 g auf einem Areal von 15 cm x 2,5 cm, entspricht 7 mg/cm²

In weiteren Vergleichsuntersuchungen können die Auftragsmengen in folgenden Bereichen gewählt werden, um eine höheren Praxisbezug zu gewährleisten, was an den gezeigten Ergebnissen jedoch keine signifikanten Änderungen ergibt.
Produkt: möglicher Bereich: 10 mg/cm² bis 50 mg/cm², vorzugsweise 13 mg/cm² bis 40 mg/cm²
Sebum: möglicher Bereich: 2 mg/cm² bis 15 mg/cm², vorzugsweise 5 mg/cm² bis 10 mg/cm²
Humanschweiß: möglicher Bereich: 5 mg/cm² bis 40 mg/cm², vorzugsweise 7 mg/cm² bis 30 mg/cm²
Verhältnis Produkt zu Sebum 1:1 bis 7:1, vorzugsweise 2:1 bis 5:1
Verhältnis Produkt zu Humanschweiß 1:3 bis 7:1, vorzugsweise 1:1 bis 4:1

### Farbmessung:

photometrisch, Ermittlung der Farbmaßzahlen im CIE- L*a*b-Farbraum
Differenzbildung gegenüber einem unverfleckten Referenzmaterial
Mittelwert aus mind. 5 Messungen pro Areal
Zur Auswertung wird vorzugsweise der b-Wert verwendet (Gelbwert)

### Ablauf:

1. Vorwaschen und Trocknen des Textilzuschnitte
2. Kennzeichnung der Areale
3. Bevorzugtes Auftragen der Medien in folgender Reihenfolge: a.) Zubereitung, b.) ggf. Schweiß, c.) Sebum, nach jedem Material mind. 10 min warten, bis es eingezogen ist
   Alternativ können die Substanzen vollständig oder teilweise vorab miteinander vermischt werden und dann in dieser Mischung aufgetragen werden.
4. Einlagern der Proben bei 38°C und 80% Luftfeuchte vorzugsweise für mind. 12 Stunden
5. Waschen der Textilproben einzeln im Linitest (z. B. 1g pulverförmiges Waschmittel auf 300 ml Leitungswasser, Waschtemperatur 60°C) und Auspülen mit kaltem Leitungswasser
6. Trocknen bei Raumtemperatur
7. Farbmessung auf den verfleckten Arealen und einem unverfleckten Referenzareal
8. Wiederholung des Ablaufes von Punkt 2 bis 7 mindestens 1 mal, vorzugsweise 4 mal

Die Methode zur Überprüfung der durch antitranspirantwirksame Substanzen enthaltende kosmetische oder dermatologische Zubereitungen mitverursachten Flecken auf oder in Kleidung ist demnach ebenso erfindungsgemäß. Es werden dabei nacheinnader
a.) Zubereitung, in einer Menge von 10 mg/cm² bis 50 mg/cm²,
b.) ggf. Humanschweiß, in einer Menge von 5 mg/cm² bis 40 mg/cm² und
c.) Sebum, in einer Menge von 2 mg/cm² bis 15 mg/cm²,
auf die gleiche Stelle der Kleidung aufgetragen. Anschließend wird
- ggf. die Kleidung bei 38°C und 80% Luftfeuchte vorzugsweise für mind. 12 Stunden eingelagert,
- die Kleidung einzeln gewaschen,
- ggf. mit kaltem Leitungswasser ausgespült,
- bei Raumtemperatur getrocknet und
photometrische die verfleckten Areale gegen unverfleckte Referenzareale der gleichen Kleidung mittels Farbmaßzahlen im CIE- L*a*b-Farbraum vermessen.

### In vivo Methode zur Bestimmung der Fleckintensität (wie zuvor beschrieben)

### Materialien:

Textil: weißes, 100% Baumwolle, T-Shirt
Waschmittel: handelsübliche Waschmittel, Persil
Waschmaschine: handelsübliches Gerät
Wasserqualität: mittelhartes bis hartes Wasser

### Farbmessung:

photometrisch, Ermittlung der Farbmaßzahlen im CIE- L*a*b-Farbraum
Differenzbildung gegenüber einem unverfleckten Referenzmaterial z. B. im Schulterbereich des T-Shirts
Mittelwert aus mind. 5 Messungen pro Areal
Zur Auswertung wird vorzugsweise der b-Wert verwendet (Gelbwert)

### Ablauf:

1. Vorwaschen der T-Shirts
2. Verteilung des Produktes / der Produkte (möglich ist jedes Produkt nacheinander oder vorzugsweise ein Direktvergleich zweier Produkte rechte Achsel vs. Linke Achsel)
3. Produktanwendung mind. 3 Tage zur Vorkondionierung
4. Tragen des T-Shirts nach Produktanwendung morgens für mind. 8 Stunden
5. Waschen des T-Shirts bei vorzugsweise 60°C (abhängig vom Textilmaterial)
6. Trocknen in einem handelsüblichen Wäschetrockner
7. Farbmessung auf den verfleckten Arealen im Achselbereich und einem unverfleckten Referenzareal
8. Wiederholung des Ablaufes von Punkt 4 bis 7 mindestens 3 mal

### Ergebnisse der in vitro Versuche (nach 4 Zyklen)

| **Produkte als Roller / Gew.%** | **Vgl. 1** | **P2** | **P3** |
|---|---|---|---|
| Aluminum Chlorhydrate | 10,0 | 10,0 | 10,0 |
| Isoceteth-20 | 4,8 | 4,8 | 4,8 |
| Dicaprylylether | 3,0 | 3,0 | 3,0 |
| Glycerylisostearat | 2,4 | 2,4 | 2,4 |
| PEG-150 Distearat | 0,7 | 0,7 | 0,7 |
| Butylenglykol | 3,0 | 3,0 | 3,0 |
| Varisoft ® PATC | | 3,0 | 3,0 |
| Plantapon LC7 | | | 1,0 |
| Wasser | 76,1 | 73,1 | 72,1 |
| *b-Wert* | *8,1* | *5,1* | *3,6* |

| **Produkte als Roller / Gew.%** | **Vgl 2** | **P4** | **P5** |
|---|---|---|---|
| Aluminum Chlorhydrate | 10,0 | 10,0 | 10,0 |
| Isoceteth-20 | 4,8 | 4,8 | 4,8 |
| Paraffinum Liquidum | 3,0 | 3,0 | 3,0 |
| Glycerylisostearat | 2,4 | 2,4 | 2,4 |
| Butylenglykol | 3,0 | 3,0 | 3,0 |
| PEG-150 Distearat | 0,7 | 0,7 | 0,7 |
| Varisoft® PATC | - | 1,0 | 2,0 |
| Plantapon LC7 | - | 1,0 | 1,0 |
| Parfüm | 1,0 | 1,0 | 1,0 |
| Wasser | ad 100 | ad 100 | ad 100 |
| *b-Wert* | *3,9* | *2,4* | *1,9* |

| | | | |
|---|---|---|---|
| Varisoft® PATC: 60% Palmitamidopropyltrimonium Chloride + 40% Propylene Glycol | | | |

| **Produkte als Stifte / Gew.%** | **Vgl 3** | **P6** |
|---|---|---|
| Aluminum Zirconium Tetrachlorohydrex GLY | 16,0 | 16,0 |
| Glycerylstearat | 0,6 | 0,6 |
| Stearylalkohol | 20,0 | 20,0 |
| Hydrogenated Castor Oil | 1,5 | 1,5 |
| PPG-14 Butylether | 15,0 | 15,0 |
| Cyclomethicone | 41,9 | 38,9 |
| Talkum | 4,0 | 4,0 |
| Varisoft® PATC | - | 3,0 |
| Parfüm | 1,0 | 1,0 |
| *b-Wert* | *7,2* | *4,9* |

| **Produkte als Stifte /Gew.%** | **Vgl 4** | **P7** | **P8** | **P9** |
|---|---|---|---|---|
| Aluminum Zirconium Tetrachlorohydrex GLY | 16 | 16 | 16 | 16 |
| Glycerylstearat | 2 | 2 | 2 | 2 |
| Stearylalkohol | 20 | 20 | 20 | 20 |
| Hydrogenated Castor Oil | 1,5 | 1,5 | 1,5 | 1,5 |
| Caprylic/Capric Triglyceride | 10 | 10 | 10 | 10 |
| Paraffinum Liquidum | 12 | 12 | 12 | 12 |
| PPG-14 Butylether | 20 | 19 | 18 | 18 |
| Cyclomethicone | 13,5 | 13,5 | 13,5 | 13,5 |
| Talkum | 4 | 4 | 4 | 4 |
| Varisoft® PATC | - | 1 | 1 | 1,5 |
| Plantapon LC 7 | - | | 1 | 0,5 |
| Parfüm | 1 | 1 | 1 | 1 |
| *b-Wert* | *5,6* | *4,3* | *3,4* | *3,6* |

| **Produkte als Aerosole /Gew.%** | **Vgl 5** | **P10** |
|---|---|---|
| Aluminum Chlorohydrate | 20 | 20 |
| Cetyl PEG/PPG-10/1 Dimethicone | 1,5 | 3,0 |
| Polyglyceryl-2 Dipolyhydroxystearate | 1,5 | - |
| Cyclomethicone | 11,75 | 11,75 |
| C12-15 Alkylbenzoate | 8 | 8 |
| Dicaprylyl Ether | 6 | 6 |
| Octyldodecanol | 8 | 8 |
| Parfüm | 3,5 | 3,5 |
| Varisoft® PATC | - | 3,0 |
| Wasser | ad 100 | ad 100 |
| *b-Wert* | *7,2* | *4,1* |

| | | |
|---|---|---|
| Aerosol Gew.% bezogen auf Emulsion, Abfüllung 30 Gew.% Emulsion : 70 Gew.% Treibgas (Gemisch aus Propan, Isobutan, Butan) | | |

### Ergebnisse der in vivo Versuche:

**10 Zyklen, 26 Probanden**

| | Vgl 6 | P11 |
|---|---|---|
| Aluminumchlorhydrat | 10,0 | 10,0 |
| Isoceteth-20 | 4,8 | 4,8 |
| Dicaprylylether | 3,0 | 3,0 |
| Glycerylisostearat | 2,4 | 2,4 |
| PEG-150 Distearat | 0,7 | 0,7 |
| Butylenglykol | 3,0 | 3,0 |
| Varisoft® PATC | | 3,0 |
| Plantapon LC7 | | 1,0 |
| Parfüm | 1,1 | 1,1 |
| Wasser | 75,0 | 71,0 |
| *b-Wert* | *0,55* | *0,38* |

Die Vergleichsuntersuchungen zeigen, dass die erfindungsgemäßen Zubereitungen P1 - P11, umfassend mindestens ein geladenes Tensid, eine verbesserte Auswaschbarkeit und verminderte Fleckenbildung gegenüber Zubereitungen ohne diese geladenen Tenside (Vgl 1 - Vgl 6) aufweisen.

Die Vergleichsuntersuchungen und die nachfolgende Beispiele verdeutlichen eindrucksvoll, dass die erfindungsgemäßen Zubereitungen, in denen der Zusatz von geladenen Tensiden zu einer Verminderung der Fleckenbildung in oder auf der Kleidung führt und zusätzlich zu einer verbesserten Auswaschbarkeit der Flecken aus der Kleidung beiträgt.

Erfindungsgemäß ist demnach das Verfahren zur Verminderung oder Vermeidung von Flecken in oder auf der Kleidung mitverursacht durch die Zubereitung, wobei das Verfahren die Schritte umfasst:
- topische Applikation einer kosmetischen oder dermatologischen Zubereitung umfassend
   ∘ ein oder mehrere antitranspirantwirksame Substanzen und ein oder mehrere geladene Tenside,
- Tragen von Kleidung über den topisch applizierten Hautbereichen und anschließendem
- Waschen der Kleidung.

Die erfindungsgemäßen Zusammensetzungen können ferner gegebenenfalls in der Kosmetik übliche Zusatzstoffe, beispielsweise Parfüm, Verdicker, Desodorantien, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate enthalten, sofern diese nicht die erfindungsgemäße Zusammensetzung und deren Verwendung zu wider läuft.

Moisturizer können vorteilhaft auch als Antifaltenwirkstoffe zum Schutz vor Hautveränderungen, wie sie z. B. bei der Hautalterung auftreten, verwendet werden
Erfindungsgemäß sind die Formulierungen vorteilhaft makroskopisch einphasig bezogen auf die flüssigen Anteile der Formulierung. Das heißt: mit dem bloßen Auge sind die in der Formel enthaltenen und ggf. miteinander vermischten Flüssigkeiten als nur als eine Phase bei Raumtemperatur erkennbar. Mikroskopisch, also in der inneren Struktur, können jedoch mehr als eine Phase vorliegen, z. B. wie bei einer Emulsion. Davon unabhängig können erfindungsgemäße Zubereitungen feste Stoffe umfassen, die ggf. eine sichtbare Phasengrenze zur einphasigen Flüssigkeit ausbilden. Diese fest-flüssig Phasengrenze zählt erfindungsgemäß zur Einphasigkeit hinzu, da sich die Einphasigkeit auf die flüssig-flüssig Bestandteile bezieht. Die makroskopisch einphasigen Systeme können damit Partikel enthalten (Suspension).

In Form einer Aerosolzubereitung sind ggf. enthaltende Treibgase nicht zu den Flüssigkeiten hinzu zuzählen, die ggf. eine Phasengrenze ausbilden können.

Diese Einphasigkeit ist ein wesentliches Unterscheidungskriterium gegenüber den Zubereitungen des Standes der Technik, wie sie beispielsweise in der DE 10321138 beschrieben sind.

Darin bedeutet das Merkmal "zweiphasig", dass die Formulierung eine wässrige und eine Ölphase aufweist, die im Ruhestand als zwei durch eine Phasengrenze getrennte Flüssigkeitsschichten vorliegen, aber vor dem Gebrauch durch kurzes Schütteln des Behälters zu einer Öl-in-Wasser-Emulsion oder einer Wasser-in-ÖI-Emulsion vermischt werden können. Jedoch hebt sich dieses Vermischen der beiden Phasen schnell und möglichst vollständig wieder auf, sobald die Zubereitung im Ruhezustand ist. Die Zeit bis zur vollständigen Entmischung reicht dabei von wenigen Sekunden bis zu 24 Stunden.

Die erfindungsgemäßen Zubereitungen weisen durch die Einphasigkeit eine homogenere Verteilung der sie beinhaltenden Bestandteile auf. Diese homogene Verteilung ist beispielsweise für die geladenen Tenside in der Zubereitung mitentscheidend für dessen erfindungsgemäße Fleckenminderung auf oder in der Kleidung.

Weiteres wesentliches Unterscheidungskriterium gegenüber DE 10321138 ist, dass keine nichtionischen Tenside und vor allem keine Emulgatoren als geladene Tenside erfindungsgemäß zu wählen sind.

Bevorzugte Applikationsformen der erfindungsgemäßen Zubereitung sind Roll-on, Stiftform oder die Aerosolform. Als Treibgas wird erfindungsgemäß bevorzugt Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Stickstoffoxide, Lachgas, 1,1,1,3 Tetrafluorethan, Heptafluoro-n-propan, Perfluorethan, Monochlordifluormethan, 1,1-Difluorethan und Kohlendioxid einzeln oder in Kombination eingesetzt. Besonders bevorzugt sind Propan, Butan, iso-Butan bzw. Mischungen dieser Treibgase.

Die genannten Gase können im Sinne der vorliegenden Erfindung jeweils einzeln oder in beliebigen Mischungen zueinander verwendet werden.

Die Zahlenangaben sind Gewichtsanteile bezogen auf die Gesamtmasse der Zubereitung.

### Beispiele

| | Beispiel 1 Zerstäuber | Beispiel 2 Roller | Beispiel 3 Roller |
|---|---|---|---|
| Aluminumchlorhydrat | 10,00 | 10,00 | 7,50 |
| Isoceteth-20 | 4,80 | 4,80 | |
| Dicaprylylether | 3,00 | 3,00 | |
| Glycerylisostearat | 2,40 | 2,40 | |
| PEG-150 Distearat | | 0,70 | |
| Butylenglykol | 3,00 | | |
| PPG-14 Butylether | | | 3,50 |
| Diethylhexylcarbonat | | | 3,50 |
| Polyglyceryl-3 Caprylat | | | 0,50 |
| Polyglyceryl-4 Laurat | | | 0,25 |
| Methylglukose Sesquistearat | | | 1,75 |
| Hydroxyethylcellulose | | | 0,60 |
| Varisoft® PATC | 3,00 | 3,00 | 5,00 |
| Parfüm | 1,00 | 1,00 | 1,00 |
| Wasser | 72,80 | ad 100 | 77,40 |

| | Beispiel 4 Creme | Beispiel 5 Stift |
|---|---|---|
| Aluminum Zirconium Tetrachlorohydrex GLY | | 16,0 |
| Aluminiumchlorhydrat | 10,0 | |
| Wasser | 64,5 | |
| Paraffinum Liquidum | 5,0 | |
| C13-16 Isoparaffin | 5,0 | |
| PEG-40 Stearat | 3,5 | |
| Trisodium EDTA | 1,5 | |
| C12-15 Alkylbenzoat | 0,5 | |
| Glycerylstearat | 3,0 | 0,6 |
| Cetylalkohol | 3,0 | |
| Stearylalkohol | | 20,0 |
| Hydrogenated Castor Oil | | 1,5 |
| PPG-14 Butylether | | 15,0 |
| Cyclomethicone | | 38,9 |
| Talkum | | 4,0 |
| Varisoft® PATC | 3,0 | 3,0 |
| Parfum | 1,0 | 1,0 |

| | | |
|---|---|---|
| Varisoft® PATC: 60% Palmitamidopropyltrimoniumchlorid, 40% Propylenglykol. | | |

| Roller/Beispiel | 6 | 7 | 8 | 9 |
|---|---|---|---|---|
| Aluminum Chlorohydrate | 8,0 | 8,0 | 8,0 | 8,0 |
| Isoceteth-20 | 5,0 | 4,8 | 4,0 | 4,0 |
| Glyceryl Isostearate | 2,2 | 2,4 | 2,0 | 2,0 |
| Dicaprylyl Ether | - | 3,0 | - | - |
| Dicaprylal Carbonate | - | - | - | 3,0 |
| Paraffinum Liquidum | 3,0 | - | 3,0 | - |
| Octyldodecanol | - | - | 3,0 | 3,0 |
| Persea Gratissima Oil | 0,1 | 0,1 | 0,1 | 0,1 |
| Butylene Glycol | 3,0 | 3,0 | - | - |
| Glycerin | - | - | 3,0 | 3,0 |
| PEG-150 Distearat | 0,7 | 0,7 | 0,7 | 0,7 |
| Palmitamidopropyltrimonium Chloride + Propylene Glycol (Varisoft® PATC) | 1,0 | 1,0 | 1,0 | 1,0 |
| Laureth-7 Citrate (Plantapon LC 7) | 1,0 | 1,0 | 1,0 | 1,0 |
| Parfüm | 1,0 | 1,0 | 1,0 | 1,0 |
| Wasser | ad 100 | ad 100 | ad 100 | ad 100 |

| Roller/Beispiel | 10 | 11 |
|---|---|---|
| Trisodium EDTA | 1,5 | 1,5 |
| Steareth-21 | 1,5 | 1,0 |
| Steareth-2 | 2,5 | 2,0 |
| PPG-15 Stearyl Ether | 3,0 | 3,0 |
| Aluminum Chlorohydrate | 10,0 | 10,0 |
| Plantapon LC 7 | 2,5 | |
| TEGO Care CE 40 | | 3,0 |
| Aqua | ad 100 | ad 100 |

| | | |
|---|---|---|
| TEGO Care CE 40: 40% Palmitamidopropyltrimonium Chloride + 60% Cetearyl Alcohol | | |

| Aerosole/Beispiel | 12 | 13 | 14 |
|---|---|---|---|
| Aluminum Chlorohydrate | 35.00 | 35.00 | 35.00 |
| Octyldodecanol | 12.00 | 12.00 | 12.00 |
| Disteardimonium Hectorite | 4.00 | 4.00 | 4.00 |
| Varisoft ® PATC | 3.00 | | |
| TEGO Care CE 40 | | 3,00 | |
| Plantapon LC 7 | | | 3,00 |
| Parfum | 6.25 | 6.25 | 6.25 |
| Cyclomethicone | ad 100 | ad 100 | ad 100 |

## Patentansprüche

1. Kosmetische oder dermatologische Zubereitung umfassend ein oder mehrere antitranspirantwirksame Substanzen und ein oder mehrere geladene Tenside **dadurch gekennzeichnet, dass** als Tensid mindestens eine Verbindung der Formel (I) enthalten ist 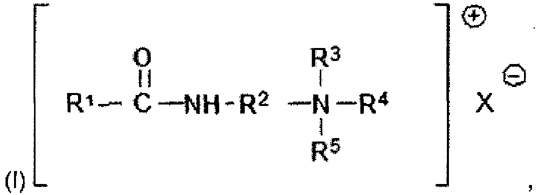 wobei
R1 für eine gesättigte oder ungesättigte, lineare oder verzweigte Alkylgruppe mit 8 bis 24 C-Atomen,
R2 für eine gesättigte oder ungesättigte, lineare oder verzweigte Alkylengruppe mit 1 bis 10 C-Atomen und 0 bis 5 Hydroxygruppen,
R3, R4, R5 unabhängig voneinander für Wasserstoff und/oder eine C1-C4-Alkylgruppe, und
X- für ein monovalentes Anion stehen.

2. Zubereitung nach Anspruch 1 **dadurch gekennzeichnet, dass** als Tensid mindestens eine Verbindung der Formel (I) gewählt wird in denen
R1 für eine gesättigte, lineare C10-C18-Alkylgruppe
R2 für die Gruppe -CH2CH2CH2-,
R3, R4 und R5 unabhängig voneinander für Methyl-, Ethyl-, Propyl-, Isopropyl- und Butylgruppen, insbesondere für Methylgruppen,
und
X- für Halogenid, insbesondere Chlorid, oder für eine der beiden Gruppen CH3-O- SO3 (Methosulfat) oder CH3CH2-O-SO3 (Ethosulfat), insbesondere Methosulfat stehen.

3. Zubereitung nach einem der vorstehenden Ansprüche **dadurch gekennzeichnet, dass** als geladenes Tensid Palmitamidopropyltrimoniumchloride gewählt wird.

4. Zubereitung nach einem der vorstehenden Ansprüche **dadurch gekennzeichnet, dass** als antitranspirantwirksame Substanzen Aluminiumverbindungen gewählt werden.

5. Zubereitung nach einem der vorstehenden Ansprüche **dadurch gekennzeichnet, dass** die Gewichtsanteile an Antitranspirantwirkstoffen, insbesondere Aluminiumchlorohydrate, im Bereich von 0,05 bis 40 Gew.%, vorzugsweise von 0,1 bis 20 Gew. %, insbesondere 1 bis 25 Gew.%, insbesondere 5 bis 20 Gew.%, bezogen auf die Gesamtmasse der Zubereitung gewählt werden.

6. Zubereitung nach einem der vorstehenden Ansprüche **dadurch gekennzeichnet, dass** mindestens ein kationisches und mindestens ein anionisches Tensid enthalten ist.

7. Zubereitung nach Anspruch 6, **dadurch gekennzeichnet, dass** als anionisches Tensid Laureth-7 Citrate gewählt wird.

8. Zubereitung nach Anspruch 6 oder 7 **dadurch gekennzeichnet, dass** das Gewichtsverhältnis zwischen kationischen Tensiden und anionischen Tensiden im Bereich 10 : 1 bis 1 : 3, vorzugsweise 6 : 1 bis 1 : 2 liegt.

9. Zubereitung nach einem der vorstehenden Ansprüche, **dadurch gekennzeichnet, dass** die Zubereitung einphasig ist.

10. Zubereitung nach einem der vorstehenden Ansprüche **dadurch gekennzeichnet, dass** Guerbet-Alkohole, insbesondere Octyldodecanol, enthalten sind.

11. Zubereitung nach einem der vorstehenden Ansprüche in einer Roll-on-, Stift- oder Aerosolform.

12. Verwendung von ein oder mehreren geladenen Tensiden in kosmetischen oder dermatologischen Zubereitungen umfassend ein oder mehrere antitanspirantwirksame Stoffe zur Verminderung oder Vermeidung der Fleckenbildung in oder auf der Kleidung **dadurch gekennzeichnet, dass** als Tensid mindestens eine Verbindung der Formel (I) gewählt wird 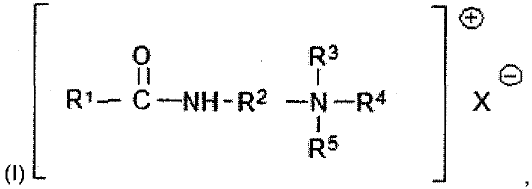 wobei
R1 für eine gesättigte oder ungesättigte, lineare oder verzweigte Alkylgruppe mit 8 bis 24 C-Atomen,
R2 für eine gesättigte oder ungesättigte, lineare oder verzweigte Alkylengruppe mit 1 bis 10 C-Atomen und 0 bis 5 Hydroxygruppen,
R3, R4, R5 unabhängig voneinander für Wasserstoff und/oder eine C1-C4-Alkylgruppe, und
X- für ein monovalentes Anion stehen.

13. Verwendung von ein oder mehreren geladenen Tensiden in kosmetischen oder dermatologischen Zubereitungen umfassend ein oder mehrere antitanspirantwirksame Stoffe zur Verbesserung der Auswaschbarkeit von durch die Zubereitung mit verursachten Flecken aus der Kleidung **dadurch gekennzeichnet, dass** als Tensid mindestens eine Verbindung der Formel (I) gewählt wird 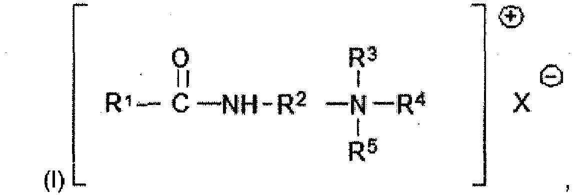 wobei
R1 für eine gesättigte oder ungesättigte, lineare oder verzweigte Alkylgruppe mit 8 bis 24 C-Atomen,
R2 für eine gesättigte oder ungesättigte, lineare oder verzweigte Alkylengruppe mit 1 bis 10 C-Atomen und 0 bis 5 Hydroxygruppen,
R3, R4, R5 unabhängig voneinander für Wasserstoff und/oder eine C1-C4-Alkylgruppe, und
X- für ein monovalentes Anion stehen.

14. Verwendung nach einem der vorstehenden Ansprüche 12 oder 13, **dadurch gekennzeichnet, dass** die Flecken nach ein oder mehrfachen Waschen einen verminderten b-Wert aufweisen, der photometrisch mittels der Farbmaßzahlen im CIE- L*a*b-Farbraum ermittelt wird.

15. Verwendung nach einem der vorstehenden Ansprüche 12 bis 14 **dadurch gekennzeichnet, dass** als geladenes Tensid Palmitamidopropyltrimonium Chloride verwendet wird.

16. Verwendung nach einem der vorstehenden Ansprüche 12 bis 15 **dadurch gekennzeichnet, dass** mindestens ein kationisches und mindestens ein anionische Tensid enthalten ist.

17. Verwendung nach Anspruch 16 **dadurch gekennzeichnet, dass** das Gewichtsverhältnis kationisches zu anionisches Tensid im Bereich 10 : 1 bis 1 : 3, vorzugsweise 6 : 1 bis 1 : 2 liegt.

18. Verwendung nach einem der vorstehenden Ansprüche 12 bis 17, **dadurch gekennzeichnet, dass** als antitranspirantwirksame Substanzen Aluminiumverbindungen gewählt werden.

19. Verwendung nach einem der vorstehenden Ansprüche 12 bis 18 **dadurch gekennzeichnet, dass** die Gewichtsanteile an Antitranspirantwirkstoffen, insbesondere Aluminiumchlorohydrate, im Bereich von 0,05 bis 40 Gew.%, vorzugsweise von 0,1 bis 20 Gew. %, insbesondere im Bereich von 1 bis 25 Gew.%, insbesondere 5 bis 20 Gew.%, bezogen auf die Gesamtmasse der Zubereitung gewählt werden.

20. Verwendung nach einem der vorstehenden Ansprüche 12 bis 19 **dadurch gekennzeichnet, dass** Guerbet-Alkohole, insbesondere Octyldodecanol, in der Zubereitung enthalten sind.

## Claims

1. Cosmetic or dermatological preparation comprising one or more antiperspirant substances and one or more charged surfactants, **characterized in that** at least one compound of the formula (I) is present as surfactant 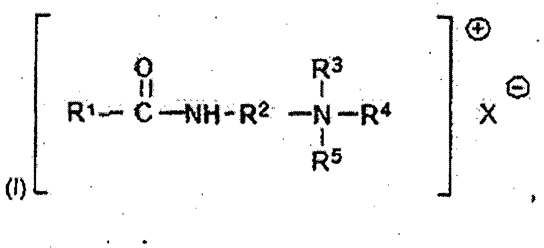 wherein
R1 is a saturated or unsaturated, linear or branched alkyl group having 8 to 24 carbon atoms,
R2 is a saturated or unsaturated, linear or branched alkylene group having 1 to 10 carbon atoms and 0 to 5 hydroxyl groups,
R3, R4, R5 are each independently hydrogen and/or a Cl-C4-alkyl group,
and
X- is a monovalent anion.

2. Preparation according to Claim 1, **characterized in that** at -least one compound of the formula (I) is selected as surfactant in which
R1 is a saturated, linear C10-C18-alkyl group
R2 is the group -CH2CH2CH2-,
R3, R4 and R5 are each independently methyl, ethyl, propyl, isopropyl and butyl groups, especially are methyl groups,
and
X- is halide, especially chloride, or is one of the two groups CH3-O-SO3 (methosulfate) or CH3CH2-O-SO3 (ethosulfate), especially methosulfate.

3. Preparation according to either of the preceding claims, **characterized in that** palmitamidopropyltrimonium chloride is selected as charged surfactant.

4. Preparation according to any of the preceding claims, **characterized in that** aluminium compounds are selected as antiperspirant substances.

5. Preparation according to any of the preceding claims, **characterized in that** the proportions by weight of antiperspirant active ingredients, especially aluminium chlorohydrates, are selected in the range of 0.05 to 40% by weight, preferably of 0.1 to 20% by weight, particularly 1 to 25% by weight, especially 5 to 20% by weight, based on the total mass of the preparation.

6. Preparation according to any of the preceding claims, **characterized in that** at least one cationic and at least one anionic surfactant is present.

7. Preparation according to Claim 6, **characterized in that** the anionic surfactant selected is laureth-7 citrate.

8. Preparation according to Claim 6 or 7, **characterized in that** the ratio by weight between cationic surfactants and anionic surfactants is in the range from 10:1 to 1:3, preferably from 6:1 to 1:2.

9. Preparation according to any of the preceding claims, **characterized in that** the preparation is monophasic.

10. Preparation according to any of the preceding claims, **characterized in that** Guerbet alcohols, especially octyldodecanol, are present.

11. Preparation according to any of the preceding claims in the form of a roll-on, pencil or aerosol.

12. Use of one or more charged surfactants in cosmetic or dermatological preparations comprising one or more antiperspirant substances for preventing or avoiding stain formation in or on clothing, **characterized in that** the surfactant selected is at least one compound of the formula (I) 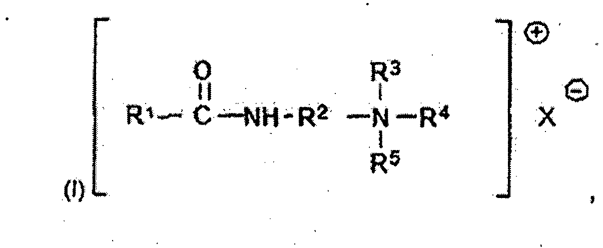 wherein
R1 is a saturated or unsaturated, linear or branched alkyl group having 8 to 24 carbon atoms,
R2 is a saturated or unsaturated, linear or branched alkylene group having 1 to 10 carbon atoms and 0 to 5 hydroxyl groups,
R3, R4, R5 are each independently hydrogen and/or a C1-C4-alkyl group,
and
X- is a monovalent anion.

13. Use of one or more charged surfactants in cosmetic or dermatological preparations comprising one or more antiperspirant substances for improving the washability of stains from clothing conjointly caused by the preparation, **characterized in that** the surfactant selected is at least one compound of the formula (I) 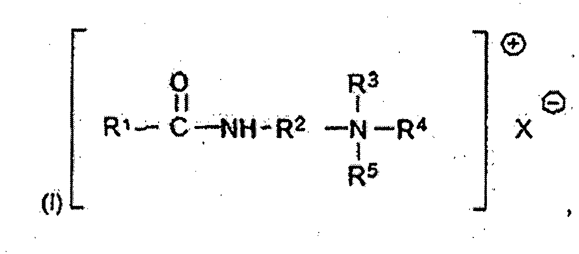 wherein
R1 is a saturated or unsaturated, linear or branched alkyl group having 8 to 24 carbon atoms,
R2 is a saturated or unsaturated, linear or branched alkylene group having 1 to 10 carbon atoms and 0 to 5 hydroxyl groups,
R3, R4, R5 are each independently hydrogen and/or a C1-C4-alkyl group,
and
X- is a monovalent anion.

14. Use according to either of preceding Claims 12 or 13, **characterized in that** the stains after one or multiple washes have a reduced b value, which is determined photometrically by means of the colourimetric values in the CIE L*a*b colour space.

15. Use according to any of preceding Claims 12 to 14, **characterized in that** palmitamidopropyltrimonium chloride is used as charged surfactant.

16. Use according to any of preceding Claims 12 to 15, **characterized in that** at least one cationic and at least one anionic surfactant is present.

17. Use according to Claim 16, **characterized in that** the ratio by weight of cationic to anionic surfactant is in the range from 10:1 to 1:3, preferably from 6:1 to 1:2.

18. Use according to any of preceding Claims 12 to 17, **characterized in that** aluminium compounds are selected as antiperspirant substances.

19. Use according to any of preceding Claims 12 to 18, **characterized in that** the proportions by weight of antiperspirant active ingredients, especially aluminium chlorohydrates, are selected in the range of 0.05 to 40% by weight, preferably of 0.1 to 20% by weight, particularly in the range of 1 to 25% by weight, especially 5 to 20% by weight, based on the total mass of the preparation.

20. Use according to any of preceding Claims 12 to 19, **characterized in that** Guerbet alcohols, especially octyldodecanol, are present in the preparation.

## Revendications

1. Préparation cosmétique ou dermatologique comprenant une ou plusieurs substances à activité antitranspirante et un ou plusieurs tensioactifs chargés, **caractérisée en ce qu'**au moins un composé de formule (I) est contenu en tant que tensioactif : 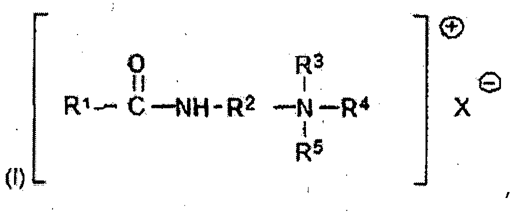 dans laquelle
R1 représente un groupe alkyle saturé ou insaturé, linéaire ou ramifié, de 8 à 24 atomes C,
R2 représente un groupe alkylène saturé ou insaturé, linéaire ou ramifié, de 1 à 10 atomes C et 0 à 5 groupes hydroxy,
R3, R4, R5 représentent indépendamment les uns des autres l'hydrogène et/ou un groupe alkyle en C1-C4, et
X⁻ représente un anion monovalent.

2. Préparation selon la revendication 1, **caractérisée en ce qu'**au moins un composé de formule (I) est choisi en tant que tensioactif, dans laquelle
R1 représente un groupe alkyle en C10-C18 linéaire saturé,
R2 représente le groupe -CH2CH2CH2-,
R3, R4 et R5 représentent indépendamment les uns des autres des groupes méthyle, éthyle, propyle, isopropyle et butyle, notamment des groupes méthyle,
et
X⁻ représente un halogénure, notamment un chlorure, ou un des deux groupes CH3-O-SO3 (méthosulfate) ou CH3CH2-O-SO3 (éthosulfate), notamment méthosulfate.

3. Préparation selon l'une quelconque des revendications précédentes, **caractérisée en ce que** le chlorure de palmitamidopropyltrimonium est choisi dans en tant que tensioactif chargé.

4. Préparation selon l'une quelconque des revendications précédentes, **caractérisée en ce que** des composés d'aluminium sont choisis dans tant que substances à activité antitranspirante.

5. Préparation selon l'une quelconque des revendications précédentes, **caractérisée en ce que** la proportion en poids d'agents actifs antitranspirants, notamment de chlorhydrate d'aluminium, est choisie dans la plage allant de 0,05 à 40 % en poids, de préférence de 0,1 à 20 % en poids, notamment de 1 à 25 % en poids, notamment de 5 à 20 % en poids, par rapport à la masse totale de la préparation.

6. Préparation selon l'une quelconque des revendications précédentes, **caractérisée en ce qu'**au moins un tensioactif cationique et au moins un tensioactif anionique sont contenus.

7. Préparation selon la revendication 6, **caractérisée en ce que** le citrate de laureth-7 est choisi en tant que tensioactif anionique.

8. Préparation selon la revendication 6 ou 7, **caractérisé en ce que** le rapport en poids entre les tensioactifs cationiques et les tensioactifs anioniques se situe dans la plage allant de 10:1 à 1:3, de préférence de 6:1 à 1:2.

9. Préparation selon l'une quelconque des revendications précédentes, **caractérisée en ce que** la préparation est monophasée.

10. Préparation selon l'une quelconque des revendications précédentes, **caractérisée en ce que** des alcools de Guerbet, notamment l'octyldodécanol, sont contenus.

11. Préparation selon l'une quelconque des revendications précédentes, sous une forme à bille, en bâton ou en aérosol.

12. Utilisation d'un ou de plusieurs tensioactifs chargés dans des préparations cosmétiques ou dermatologiques comprenant une ou plusieurs substances à activité antitranspirante pour réduire ou éviter la formation de taches dans ou sur des vêtements, **caractérisée en ce qu'**au moins un composé de formule (I) est choisi en tant que tensioactif : 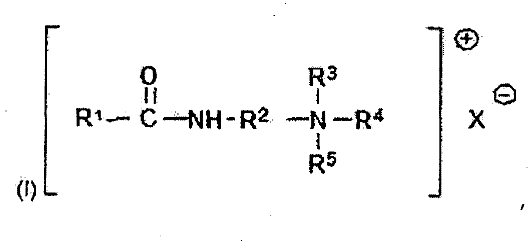 dans laquelle
R1 représente un groupe alkyle saturé ou insaturé, linéaire ou ramifié, de 8 à 24 atomes C,
R2 représente un groupe alkylène saturé ou insaturé, linéaire ou ramifié, de 1 à 10 atomes C et 0 à 5 groupes hydroxy,
R3, R4, R5 représentent- indépendamment les uns des autres l'hydrogène et/ou un groupe alkyle en C1-C4, et
X⁻ représente un anion monovalent.

13. Utilisation d'un ou de plusieurs tensioactifs chargés dans des préparations cosmétiques ou dermatologiques comprenant une ou plusieurs substances à activité antitranspirante pour améliorer l'aptitude au lavage de taches causées par la préparation sur des vêtements, **caractérisée en ce qu'**au moins un composé de formule (I) est choisi en tant que tensioactif : 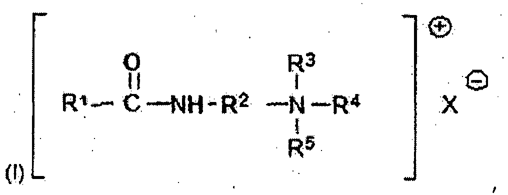 dans laquelle
R1 représente un groupe alkyle saturé ou insaturé, linéaire ou ramifié, de 8 à 24 atomes C,
R2 représente un groupe alkylène saturé ou insaturé, linéaire ou ramifié, de 1 à 10 atomes C et 0 à 5 groupes hydroxy,
R3, R4, R5 représentent indépendamment les uns des autres l'hydrogène et/ou un groupe alkyle en C1-C4, et
X⁻ représente un anion monovalent.

14. Utilisation selon l'une quelconque des revendications 12 ou 13 précédentes, **caractérisée en ce que** les taches présentent après un ou plusieurs lavages une valeur b réduite, qui est déterminée par photométrie au moyen des indices de couleur dans l'espace de couleur L*a*b CIE.

15. Utilisation selon l'une quelconque des revendications 12 à 14 précédentes, **caractérisée en ce que** le chlorure de palmitamidopropyltrimonium est utilisé en tant que tensioactif chargé.

16. Utilisation selon l'une quelconque des revendications 12 à 15 précédentes, **caractérisée en ce qu'**au moins un tensioactif cationique et au moins un tensioactif anionique sont contenus.

17. Utilisation selon la revendication 16, **caractérisée en ce que** le rapport en poids entre le tensioactif cationique et le tensioactif anionique se situe dans la plage allant de 10:1 à 1:3, de préférence de 6:1 à 1:2.

18. Utilisation selon l'une quelconque des revendications 12 à 17 précédentes, **caractérisée en ce que** des composés d'aluminium sont choisis en tant que substances à activité antitranspirante.

19. Utilisation selon l'une quelconque des revendications 12 à 18 précédentes, **caractérisée en ce que** la proportion en poids d'agents actifs antitranspirants, notamment de chlorhydrate d'aluminium, est choisie dans la plage allant de 0,05 à 40 % en poids, de préférence de 0,1 à 20 % en poids, notamment dans la plage allant de 1 à 25 % en poids, notamment de 5 à 20 % en poids, par rapport à la masse totale de la préparation.

20. Utilisation selon l'une quelconque des revendications 12 à 19 précédentes, **caractérisée en ce que** des alcools de Guerbet, notamment l'octyldodécanol, sont contenus dans la préparation.
